# EUROPÄISCHE PATENTSCHRIFT

(11) **EP 0 958 271 B1**
(45) Veröffentlichungstag und Bekanntmachung des Hinweises auf die Patenterteilung: **28.05.2003**
(21) Anmeldenummer: 97954759.3
(22) Anmeldetag: 30.12.1997
(51) Int. Cl.: C07C 45/50, C07C 47/02

(54) **VERFAHREN ZUR HERSTELLUNG VON ALDEHYDEN DURCH HYDROFORMYLIERUNG**
PROCESS FOR PREPARING ALDEHYDES BY HYDROFORMYLATION
PROCEDE DE PREPARATION D'ALDEHYDES PAR HYDROFORMYLATION

(30) Priorität: 13.01.1997 DE 19700805
(43) Veröffentlichungstag der Anmeldung: 24.11.1999
(60) Teilanmeldung: 01122277.5
(73) Patentinhaber: Celanese Chemicals Europe GmbH, 60439 Frankfurt am Main (DE)
(72) Erfinder: BOGDANOVIC, Sandra, D-65929 Frankfurt am Main (DE); BAHRMANN, Helmut, D-46499 Hamminkeln (DE); FROHNING, Carl-Dieter, D-46485 Wesel (DE); WIEBUS, Ernst, D-46147 Oberhausen (DE)
(86) Internationale Anmeldenummer: EP9707315
(87) Internationale Veröffentlichungsnummer: WO98030527

(56) Entgegenhaltungen:
- EP-A- 0 157 316
- FR-A- 2 314 910
- FR-A- 2 489 308
- US-A- 3 404 188

## Beschreibung

Die vorliegende Erfindung betrifft ein Verfahren zur Herstellung von Aldehyden durch Umsetzung von Propen mit Wasserstoff und Kohlenmonoxid bei erhöhtem Druck in Anwesenheit einer Rhodium und sulfonierte Triarylphosphine als Katalysator enthaltenden wäßrigen Phase.

Es ist bekannt, durch Umsetzung von Olefinen mit Kohlenmonoxid und Wasserstoff Aldehyde und Alkohole herzustellen. Die Reaktion wird durch Hydridometaflcarbonyle, vorzugsweise solcher der Metalle der 8. Gruppe des Periodensystems, katalysiert. Neben Kobalt, das als Katalysatormetall in großem Umfang technische Anwendung findet, hat in letzter Zeit Rhodium zunehmende Bedeutung erlangt. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls im Überschuß eingesetzte Liganden enthalten. Besonders bewährt haben sich als Liganden tertiäre Phosphine oder Phosphite. Ihre Anwendung ermöglicht, den Reaktionsdruck auf Werte unter 30 MPa zu senken.

Probleme wirft bei diesem Verfahren jedoch die Abtrennung der Reaktionsprodukte und die Wiedergewinnung der im Reaktionsprodukt homogenen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 5 Kohlenstoffatomen im Molekül, beschritten werden.

Bei der Hydroformylierung langkettiger Olefine oder olefinischer Verbindungen mit funktionellen Gruppen werden Produkte mit hohem Siedepunkt gebildet, die sich destillativ vom homogen gelösten Rhodium-Komplexkatalysator nicht abtrennen lassen. Die thermische Belastung des Destillationsgutes führt durch Dickölbildung zu erheblichen Verlusten an Wertprodukten und an Katalysator durch Zersetzung der Rhodiumkomplexverbindungen.

Die Abtrennung des Katalysators auf thermischem Wege wird durch Anwendung in Wasser löslicher Katalysatorsysteme vermieden. Derartige Katalysatoren sind z.B. in der DE-PS 26 27 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d.h. ohne Destillation und damit ohne zusätzliche thermische Verfahrensschritte. Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Neben sulfonierten Triarlyphosphinen werden als Komplexbestandteile wasserlöslicher Rhodiumkomplexverbindungen auch carboxylierte Triarylphosphine eingesetzt.

Der Einsatz wasserlöslicher Katalysatoren hat sich bei der Hydroformylierung niederer Olefine, insbesondere Propen und Buten, bewährt. Setzt man höhere Olefine wie Penten oder Hexen ein, geht jedoch die Umsetzungsgeschwindigkeit bereits merklich zurück. Die Wirtschaftlichkeit der Umsetzung in technischem Maßstab ist bei Einsatz von Olefinen mit mehr als vier Kohlenstoffatomen häufig nicht mehr im gewünschten Umfang gegeben.

Aus der EP-A1-0 805 139 ist ein Hydroformylierungsverfahren für höhere Olefine bekannt, das in einem polaren organischen Lösungsmittel, beispielsweise in einem Polyalkylenglykol, in Gegenwart von mono-sulfonierten tertiären organischen Phosphinen durchgeführt wird. Nach erfolgter Hydroformylierungsreaktion wird das Rohprodukt mit Wasser extrahiert, wobei das organische Reaktionsprodukt zurückbleibt und die Rhodiumverbindung, das mono-sulfonierte Phosphin sowie das polare organische Lösungsmittel in die wäßrige Phase übergehen. Nach der Wasserentfernung und dem Ansäuern bleibt das polare, organische Lösungsmittel, enthaltend die Rhodiumverbindung und das mono-sulfonierte Phosphin, zurück, das anschließend in den Reaktor zurückgefahren werden kann.

Um bei der Hydroformylierung höherer Olefine mittels wasserlöslicher Katalysatoren den Umsatz und/oder die Selektivität der Reaktion zu n-Aldehyden zu steigern, wird der Zusatz eines amphiphilen Reagenz (DE 31 35 127 A1) oder eines Lösungsvermittlers (DE 34 12 335 A1) empfohlen.

Sehr hohe Umsätze werden sowohl gemäß DE 31 35 127 A1 als auch gemäß DE 34 12 335 unter Verwendung quaternärer Ammoniumsalze, die einen langkettigen Alkylrest aufweisen, erhalten, während nichtionische Stoffe auf Basis Polyethylenglykol zu vergleichsweise niedrigen Umsätzen führen.

Wie aus der DE 31 35 127 Tabelle 7 hervorgeht, führt die Hydroformylierung von Dodecen-(1) mittels Rhodium und monosulfoniertem Triphenylphosphin (3-Ph₂PC₆H₄SO₃Na) ohne Zusatz eines amphiphilen Reagenz zu einem Umsatz von 56 % (Beispiel 77), während der Zusatz von C₁₂H₂₅(OCH₂CH₂)₂₃OH (="Brij 35") zu einer Minderung des Umsatzes auf 37 % (Beispiel 78) führt.

Gemäß DE 34 12 335 (Tabelle 4) führt die Hydroformylierung von Hexen mittels Rhodium und Trinatrium-tri(n-sulfophenyl)phosphin ohne Zusatz eines Lösungsvermittlers zu einem Umsatz von 36 % (Beispiel 10), während ein Zusatz von 2,5 % Triethylenglykol (Beispiel 14) bzw. von 5 % Polyglykol 200 (Beispiel 11) einen Umsatz von 43,5 % bzw. 43 % ergibt. Der Zusatz der Lösungsvermittler hat keine signifikante Steigerung des Umsatzes zur Folge und auch die Erhöhung der Menge von 2,5 % und 5 % Lösungsvermittler bewirkt keine Erhöhung des Umsatzes. Ein sehr hoher Umsatz, nämlich 86 %, wird hingegen mit einem Zusatz von 2,5 % Trimethylhexadecylammoniumbromid erzielt.

Der Einsatz quatemärer Ammoniumsalze als amphiphiles Reagenz oder Lösungsvermittler ist allerdings wegen der schlechten biologischen Abbaubarkeit dieser Verbindungen nicht unproblematisch. So führt die Anwesenheit quaternärer Ammoniumsalze im Abwasser zu Schwierigkeiten bei Abwasseraufarbeitung.

Amphiphile Reagenzien und Lösungsvermittler dienen dazu, den Stofftransport zwischen den einzelnen Phasen und damit die Vermischbarkeit von wäßriger Katalysatorphase und organischer Phase zu begünstigen. Eine Steigerung der Vermischbarkeit von wäßriger Katalysatorphase und organischer Phase geht mit einer erhöhten Löslichkeit von organischer Phase in wäßriger Phase und von wäßriger Phase in organischer Phase einher. Auf diese Weise kann in zunehmendem Maße sowohl amphiphiles Reagenz und Lösungsvermittler als auch Rhodium und wasserlösliches Phosphin in die organische Phase gelangen und nach Phasentrennung mit der organischen Phase ausgetragen werden.

Ferner ist zu erwarten, daß mit steigender Vermischbarkeit von wäßriger Katalysatorphase und organischer Phase die für die Phasentrennung erforderliche Entmischung infolge der Bildung von Emulsionen oder Lösungen nicht mehr oder nicht in genügendem Umfange stattfindet. Eine entsprechende Steigerung der Vermischbarkeit ist insbesondere bei einer Erhöhung des Zusatzes amphiphiler Reagenzein und Lösungsvermittler zu erwarten.

Ein erhöhter Austrag von Rhodium, wasserlöslichem Phosphin und amphiphilem Reagenz oder Lösungsvermittler über die organische Phase ist ebenso wie eine herabgesetzte Entmischbarkeit der Phasen unerwünscht, da Rhodium, wasserlösliches Phosphin und amphiphiles Reagenz oder Lösungsvermittler in der wäßrigen Katalysatorphase verbleiben sollen und eine gute Entmischbarkeit eine unabdingbare Vorausetzung für die erforderliche, die Hydroformylierung abschließende Trennung von organischer und wäßriger Phase ist.

Im Hinblick auf die voranstehenden Darlegungen besteht ein Bedarf, ein Verfahren bereitzustellen, das die vorstehend genannten Nachteile vermeidet und sich zudem auf einfache Weise technisch realisieren läßt.

Diese Aufgabe wird gelöst durch ein Verfahren zur Herstellung von Aldehyden. Es ist dadurch gekennzeichnet, daß man Propen in Anwesenheit einer Rhodium und sulfonierte Triarylphosphine als Katalysator und von 1 bis 15 Gew.-% einer Verbindung der Formel (1) R(OCH₂CH₂)ₙOR¹ enthaltenden wäßrigen Phase, wobei die Triarylphosphine wenigstens drei -(SO₃)M-Reste enthalten, bei denen M gleich oder verschieden ist und M für H, ein Alkalimetallion, ein Ammoniumion, ein quatemäres Ammoniumion, ein ½ Erdalkalimetallion oder ½ Zinkion steht, und wobei in der Formel (1) R für Wasserstoff, einen geradkettigen oder verzweigten Alkylrest mit 1 bis 4 Kohlenstoffatomen oder einen Hydroxyalkylrest mit 1 bis 4 Kohlenstoffatomen, R¹ für Wasserstoff oder einen Methylrest und n für eine ganze Zahl von 3 bis 50 steht, mit Wasserstoff und Kohlenmonoxid bei 20 bis 170°C und 1 bis 300 bar umsetzt.

Hydroformyliert man Propen in Anwesenheit einer Rhodium und trisulfoniertes Triphenylphosphin enthaltenden wäßrigen Phase, so erhält man in Abwesenheit eines amphiphilen Reagenz oder Lösungsvermittlers eine recht hohe Umsetzungsgeschwindigkeit.

In Hinblick hierauf ist es überraschend, daß ein vergleichsweise geringer Zusatz von Verbindungen der Formel (1) zu einer merklichen Steigerung der bereits recht hohen Propylen-Umsetzungsgeschwindigkeit führt. Es war ferner nicht zu erwarten, daß trotz dieser Umsatzsteigerung das Verhältnis der Bildung von n-Butanal zu iso-Butanal nur sehr geringfügig beeinflußt wird. Die Bildung von n-Butanal verringert sich gegenüber der Fahrweise ohne Zusatz von Verbindungen der Formel (1) lediglich um einen recht kleinen Betrag.

Unerwartet ist auch, daß in Hinblick auf den großen Einfluß, den bereits ein vergleichsweise geringer Zusatz von Verbindungen der Formel (1) auf den Propylen-Umsatz ausübt, sowohl Rhodium und das sulfonierte Triarylphosphin als auch die Verbindung der Formel (1) fast vollständig in der wäßrigen Phase verbleiben und nicht in die organische Phase gelangen und nicht über diese aus der wäßrigen Phase ausgetragen werden.

Darüber hinaus war nicht zu erwarten, daß trotz der vergleichsweise großen Mengen von Verbindungen der Formel (1), die Entmischbarkeit von organischer Phase und wäßriger Katalysatorphase so hoch ist, daß eine schnelle Trennung von organischer Phase und wäßriger Katalysatorphase gewährleistet ist. Überraschenderweise werden schwertrennbare Emulsionen oder homogene, nicht trennbare Phasen respektive Lösungen nicht gebildet.

Die den Katalysator und die Verbindung der Formel (1) R(OCH₂CH₂)OR¹ enthaltende wäßrige Phase läßt sich auf vergleichsweise einfache Art herstellen, indem man ein wasserlösliches Rhodiumsalz, die sulfonierten Triarylphosphine und die Verbindung der Formel (1) in Wasser löst.

Geeignete Rhodiumsalze sind, ohne Anspruch auf Vollständigkeit zu erheben: Rhodium(III)sulfat, Rhodium(III)nitrat, Rhodium(III)carboxylate wie Rhodiumacetat, Rhodiumpropionat, Rhodiumbutyrat, Rhodium-2-ethylhexanoat.

Man kann die wäßrige Phase unmittelbar in die Hydroformylierung einsetzen oder sie zuvor einer Präformierung des Katalysators unter Reaktionsbedingungen unterwerfen und sie anschließend in präformierter Form zu verwenden.

Unter sulfonierten Triarylphosphinen werden Phosphine mit einem oder zwei Phosphoratomen verstanden, die je Phosphoratom drei Arylreste besitzen, die Arylreste gleich oder verschieden sind und für einen Phenyl-, Naphthyl-, Biphenyl-, Phenylnaphthyl- oder Binaphthyl-Rest, insbesondere Phenyl-, Biphenyl oder Binaphthyl-Rest stehen, die Arylreste mit dem Phosphoratom direkt oder über eine -(CH₂)ₓ-Gruppe, worin x für eine ganze Zahl von 1 bis 4, insbesondere 1 bis 2, bevorzugt 1 steht, verbunden sind, und wenigstens drei -(SO₃)M Reste enthalten, worin M gleich oder verschieden ist und für H, ein Alkalimetallion, ein Ammoniumion, ein quatemäres Ammoniumion, ein ½ Erdalkalimetallion oder ½ Zinkion, insbesondere ein Alkalimetallion, ein Ammoniumion oder quatemäres Ammoniumion, bevorzugt ein Alkalimetallion steht. Die -SO₃M Reste sitzen üblicherweise als Substituenten an den Arylresten und verleihen den Triarylphosphinen die erforderliche Wasserlöslichkeit.

Man verwendet als sulfonierte Triarylphosphine mit einem Phosphoratom, Verbindungen der Formel (2) 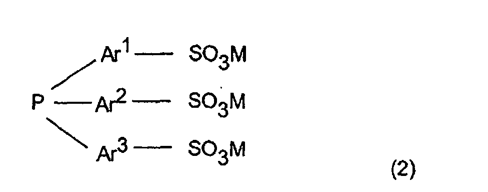 worin Ar¹, Ar² und Ar³ gleich oder verschieden sind und jeweils einen Phenyl- oder Naphthylrest, insbesondere einen Phenylrest, bedeuten, und M gleich oder verschieden, insbesondere gleich ist und für ein Alkalimetallion, ein Ammoniumion, ein quatemäres Ammoniumion oder ein 1/2 Erdkakalimetallion oder 1/2 Zinkion, insbesondere ein Akalimetallion oder Ammoniumion, bevorzugt ein Alkalimetallion, besonders bevorzugt ein Natriumion, steht.

Besonders geeignet ist Trinatrium-tri-(m-sulfophenyl)phosphin als sulfoniertes Triarylphosphin. Dieses Trinatriumsalz des Tri-(meta-sulfophenyl)phosphins enthält aufgrund seiner Herstellung durch Sulfonierung von Triphenylphosphin auch noch Anteile an mono- und disulfonierten Verbindungen.

Das Trinatrium-tri(m-sulfophenyl)phosphin entspricht der nachfolgenden Formel:

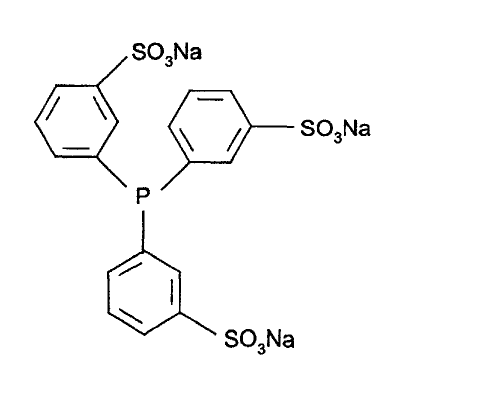

Die sulfonierten Triarylphosphine mit zwei Phosphoratomen können beispielsweise einen Rest -(CH₂)ₓ-Ar-Ar-(CH₂)ₓ- enthalten, worin x für eine ganze Zahl von 1 bis 4, insbesondere 1 bis 2, vorzugweise 1 steht, Ar-Ar Biphenyl oder Binaphthyl bedeutet, die -(CH₂)ₓ-Gruppe mit der einen Bindung jeweils in ortho-Stellung zu der die beiden Arylreste verbindenden Aryl-Arylbindung Ar-Ar steht und mit der anderen Bindung jeweils mit einem Phosphoratom, das jeweils zwei weitere, gleiche oder verschiedene Arylreste, insbesondere Phenylreste besitzt, verbunden ist. Diese zwei Phosphoratome enthaltenden Triarylphosphine weisen wenigstens drei -SO₃M Reste, insbesondere vier bis acht -SO₃M Reste, auf, worin M die vorstehend genannte Bedeutung besitzt Die -SO₃M Reste sitzen üblicherweise an den Arylresten des Restes -(CH₂)ₓ-Ar-Ar-(CH₂)ₓ- und an den zwei weiteren Arylresten, die mit dem Phosphor verbunden sind.

Beispiele für derartige, zwei Phosphoratome enthaltende, sulfonierte Triarylphosphine sind, ohne Anspruch auf Vollständigkeit zu erheben, durch die nachfolgenden Formeln (3) und (4) gegeben:

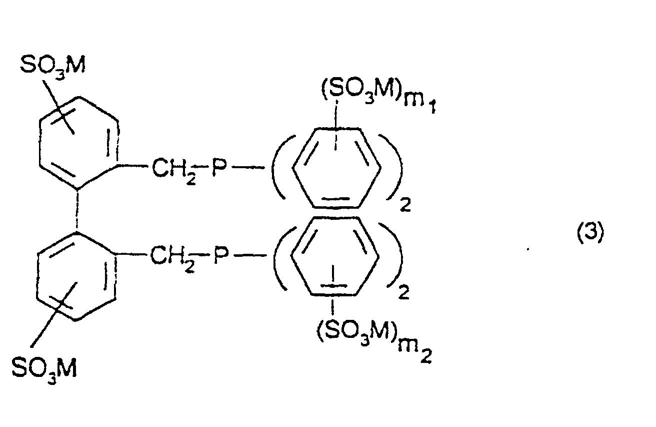

In (3) steht ein jedes m₁ und m₂ unabhängig voneinander für 0 oder 1, wobei die Verbindung der Formel (3) drei bis sechs -SO₃M Gruppen enthält.

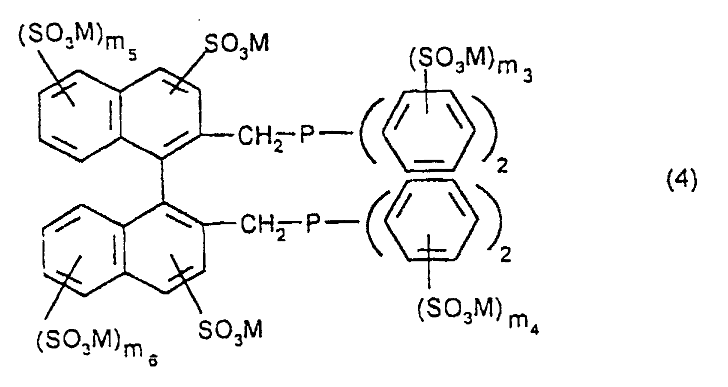

In (4) steht ein jedes m₃, m₄, m₅ und m₆ unabhängig voneinander für 0 oder 1, wobei die Verbindung der Formel (4) vier bis acht, insbesondere fünf bis sechs, -SO₃M Gruppen enthält.

Aufgrund der Herstellung durch Sulfonierung der entsprechenden Phosphine der Formel (3a) und (4a), die keine -SO₃M Gruppen enthalten, 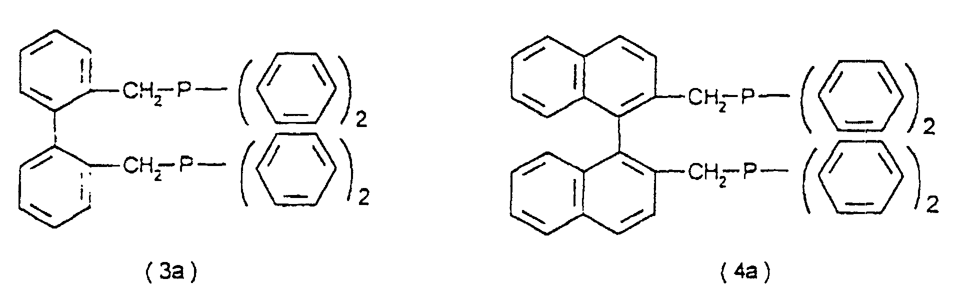 erhält man üblicherweise Gemische von Verbindungen (3) und (4) mit unterschiedlicher Anzahl von -SO₃M Gruppen. So enthält eine Verbindung der Formel (3) oder (4), die beispielsweise drei SO₃M Gruppen enthält, auch Verbindungen mit lediglich zwei -SO₃M Gruppen aber auch Verbindungen mit vier oder fünf -SO₃M Gruppen. Eine Verbindung der Formel (3) oder (4) mit beispielsweise fünf -SO₃M Gruppen enthält üblicherweise auch Verbindungen mit lediglich drei oder vier -SO₃M Gruppen, aber auch Verbindungen mit sechs oder sieben -SO₃M Gruppen.

Verbindungen der Formel (3) besitzen maximal sechs -SO₃M Gruppen, während Verbindungen der Formel (4) maximal acht -SO₃M Gruppen aufweisen.

Aus diesem Grund gelangen in der Regel Mischungen von Verbindungen der Verbindungen (3) und (4) mit unterschiedlicher Anzahl von -SO₃M Gruppen zum Einsatz.

Die vorstehend beschriebenen sulfonierten Triarylphosphine besitzen aufgrund ihrer Sulfonat-Reste eine für die Durchführung des Verfahrens ausreichende Löslichkeit in Wasser.

Man setzt die Rhodium und die Verbindungen der Formel (2) als Katalysator und die Verbindung der Formel (1) enthaltende wäßrige Phase üblicherweise entsprechend einer Menge von 2x10⁻⁶ bis 5x10⁻², insbesondere 5x10⁻⁵ bis 5x10⁻², bevorzugt 1x10⁻⁴ bis 1x10⁻³ Mol Rhodium pro Mol olefinischer Verbindung ein.

Die Rhodiummenge hängt auch von der Art der zu hydroformylierenden olefinischen Verbindung ab. Obgleich niedrigere Katalysatorkonzentrationen möglich sind, können sie sich im Einzelfall als nicht besonders zweckmäßig erweisen, da die Reaktionsgeschwindigkeit zu gering und daher nicht wirtschaftlich genug sein kann. Die obere Katalysatorkonzentration kann bis 1x10⁻¹ Mol Rhodium pro Mol olefinische Verbindung betragen. Durch vergleichsweise hohe Rhodiumkonzentrationen ergeben sich alterdings keine besonderen Vorteile.

Man setzt die Rhodium und sulfonierte Triarylphosphine als Katalysator und die Verbindung der Formel (1) R(OCH₂CH₂)ₙOR¹ enthaltende wäßrige Phase und die olefinische Verbindung üblicherweise im Volumenverhältnis 10:1 bis 1:10, insbesondere 5:1 bis 1:5, bevorzugt 2:1 bis 1:2 ein.

Man setzt Rhodium und sulfonierte Triarylphosphine im Molverhältnis 1:5 bis 1:2000 ein.

Verwendet man ein sulfoniertes Triarylphosphin mit einem Phosphoratom, beispielsweise eine Verbindung der Formel (2), so setzt man üblicherweise Rhodium und sulfoniertes Triarylphosphin im Molverhältnis 1:10 bis 1:1000, insbesondere 1:50 bis 1:200, bevorzugt 1:80 bis 1:120, ein.

Verwendet man ein sulfoniertes Triarylphosphin mit zwei Phosphoratomen (beispielsweise Verbindungen der Formel (3) und (4)), so setzt man Rhodium und sulfoniertes Triarylphosphin üblicherweise im Molverhältnis 1:5 bis 1:100, insbesondere 1:5 bis 1:50, bevorzugt 1:8 bis 1:15, ein.

Die wäßrige Phase enthält 20 bis 2000 ppm Rhodium. Falls man ein sulfoniertes Triarylphosphin mit einem Phosphoratom, beispielsweise Verbindungen der Formel (2), einsetzt, so verwendet man in den meisten Fällen eine wäßrige Phase, die 100 bis 1000, insbesondere 200 bis 500, bevorzugt 300 bis 400 ppm Rhodium enthält. Falls man ein sulfoniertes Triarylphosphin mit zwei Phosphoratomen, beispielsweise Verbindungen der Formel (3) und/oder (4) einsetzt, so verwendet man in den meisten Fällen eine wäßrige Phase, die 20 bis 500, insbesondere 30 bis 150, bevorzugt 40 bis 100 ppm Rhodium enthält.

Die Umsetzung von Propen wird in Anwesenheit einer 1 bis 15, insbesondere 3 bis 10 Gew.-% der Verbindung der Formel (1) enthaltenden wäßrigen Phase durchgeführt.

An dieser Stelle sei der Vollständigkeit halber erwähnt, daß es sich bei den Verbindungen der Formel (1) R(OCH₂CH₂)ₙOR¹, worin R für Wasserstoff, einen geradkettigen oder verzweigten Alkylrest mit 1 bis 4 Kohlenstoffatomen oder einen Hydroxyalkylrest mit 1 bis 4 Kohlenstoffatomen, insbesondere für Wasserstoff, einen Alkylrest mit 1 bis 2 Kohlenstoffatomen oder einen Hydroxyalkylrest mit 1 bis 3 Kohlenstoffatomen, bevorzugt für Wasserstoff, Methyl, Hydroxymethyl oder Hydroxypropyl und R¹ für Wasserstoff oder einen Methylrest, insbesondere Wasserstoff, steht, um Stoffe handelt, die sich in Wasser in ausreichendem Umfange lösen.

Es sei an dieser Stelle auf die nachfolgenden Verbindungen der Formel (1), worin R¹ für Wasserstoff steht, hingewiesen, die von besonderem Interesse sind.

Ohne Anspruch auf Vollständigkeit zu erheben, seien als Verbindungen der Formel R(OCH₂CH₂)ₙOH, Polyethylenglykol der Formel H(OCH₂CH₂)ₙOH mit einem mittleren Molgewicht von ungefähr 200 (PEG 200), 400 (PEG 400), 600 (PEG 600) oder 1000 (PEG 1000), Verbindungen der Formel CH₃(OCH₂CH₂)ₙOH mit einem mittleren Molgewicht von ungefähr 350 (M 350), 500 (M 500) oder 750 (M 750) oder Verbindungen der Formel CH₃CHOHCH₂(OCH₂CH₂)ₙOH mit einem mittleren Molgewicht von ungefähr 300 (300 PR), 450 (450 PR), 600 (600 PR) oder 1000 (1000PR), insbesondere Polyethylenglykol mit einem mittleren Molgewicht von ungefähr 400 (PEG 400) und 600 (PEG 600), eine Verbindung der Formel CH₃(OCH₂CH₂)ₙOH mit einem mittleren Molgewicht von 500 (M 500) oder eine Verbindung der Formel CH₃CHOHCH₂(OCH₂CH₂)ₙOH mit einem mittleren Molgewicht von 450 (450 PR) und 600 (600 PR) genannt.

Es ist unter PEG 200 ein Gemisch von Polyethylenglykolen der Formel H(OCH₂CH₂)ₙOH, worin n für eine ganze Zahl von 3 bis 6 steht, unter PEG 400 ein Gemisch von Polyethylenglykolen der Formel H(OCH₂CH₂)ₙOH, worin n für eine ganze Zahl von 7 bis 10 steht, unter PEG 600 ein Gemisch von Polyethylenglykolen der Formel H(OCH₂CH₂)ₙOH, worin n für eine ganze Zahl von 11 bis 16 steht und unter PEG 1000 ein Gemisch von Polyethylenglykolen der Formel H(OCH₂CH₂)ₙOH, worin n für eine ganze Zahl von 15 bis 30 steht, zu verstehen. Diesen Gemischen ist jeweils ein entsprechendes mittleres Molgewicht von etwa 200 (PEG 200), etwa 400 (PEG 400), etwa 600 (PEG 600) respektive etwa 1000 zuzuordnen.

Es ist unter M 350 ein Gemisch von Verbindungen der Formel CH₃(OCH₂CH₂)ₙOH, worin n für eine ganze Zahl von 5 bis 9 steht, unter M 500 ein Gemisch von Verbindungen der Formel CH₃(OCH₂CH₂)ₙOH, worin n für eine ganze Zahl von 9 bis 13 steht, unter M 750 ein Gemisch von Verbindungen der Formel CH₃(OCH₂CH₂)ₙOH, worin n für eine ganze Zahl von 12 bis 20 steht, zu verstehen. Diesen Gemischen ist jeweils ein entsprechendes mittleres Molgewicht von etwa 350 (M 350), etwa 500 (M 500) respektive 750 (M 750) zuzuordnen.

Es ist unter 300 PR ein Gemisch von Verbindungen der Formel R(OCH₂CH₂)ₙOH, worin R für einen β-Hydroxypropylrest CH₃CHOHCH₂- und n für eine ganze Zahl von 6 bis 9 steht, unter 450 PR ein Gemisch von Verbindungen der Formel R(OCH₂CH₂)ₙOH, worin R für einen β-Hydroxypropylrest CH₃CHOHCH₂- und n für eine ganze Zahl von 8 bis 14 steht, unter 600 PR ein Gemisch von Verbindungen der Formel R(OCH₂CH₂)ₙOH, worin R für einen β-Hydroxypropylrest CH₃CHOHCH₂- und n für eine ganze Zahl von 12 bis 20 steht, unter 1000 PR ein Gemisch von Verbindungen der Formel R(OCH₂CH₂)ₙOH, worin R für einen β-Hydroxypropylrest CH₃CHOHCH₂- und n für eine ganze Zahl von 18 bis 26 steht, zu verstehen. Diesen Gemischen ist jeweils ein entsprechendes mittleres Molgewicht von etwa 300 (300 PR), etwa 450 (450 PR), etwa 600 (600 PR) respektive etwa 1000 (1000 PR) zuzuordnen.

In einer Reihe von Fällen hat es sich bewährt, ein Polyethylenglykol der Formel H(OCH₂CH₂)ₙOH, worin n für eine ganze Zahl von 3 bis 50, insbesondere 4 bis 30, bevorzugt 5 bis 20, besonders bevorzugt 6 bis 12, steht, als Verbindung der Formel (1) einzusetzen.

Es hat sich auch bewährt, eine Verbindung (Halbether) der Formel R(OCH₂CH₂)ₙOH, worin R für einen Methylrest oder β-Hydroxypropylrest und n für eine ganze Zahl von 3 bis 50, insbesondere 4 bis 30, bevorzugt 5 bis 20 steht, als Verbindung der Formel (1) einzusetzen.

Es lassen sich auch beliebige Mischungen der Verbindungen der Formel (1), nämlich Polyethylenglykole, Polyethylenglykolether (Halbether) und Polyethylenglykoldiether einsetzen.

Man führt die Urnsetzung in Gegenwart von Wasserstoff und Kohlenmonoxid durch. Das Molverhältnis von Wasserstoff zu Kohlenmonoxid kann in weiten Grenzen gewählt werden und liegt üblicherweise bei 1:10 bis 10:1, insbesondere 5:1 bis 1:5, bevorzugt 2:1 bis 1:2, besonders bevorzugt 1,2:1 bis 1:1,2. Besonders einfach gestaltet sich das Verfahren, wenn man Wasserstoff und Kohlenmonoxid im Molverhältnis 1:1 oder annähernd 1:1 einsetzt.

Für viele Fälle reicht es aus, die Umsetzung bei einer Temperatur von 50 bis 150, inbesondere 100 bis 140°C durchzuführen.

In einer Vielzahl von Fällen hat es sich als nützlich erwiesen, die Umsetzung bei einem Druck von 10 bis 200, insbesondere 20 bis 150, bevorzugt 30 bis 80 bar durchzuführen.

Während der Umsetzung ist sicherzustellen, daß für eine gute Durchmischung von organischer Phase, wäßriger Phase und Kohlenmonoxid/Wasserstoff gesorgt wird. Dies kann beispielsweise durch intensives Rühren und/oder Umpumpen von organischer und wäßriger Phase bewirkt werden. In der organischen Phase befinden sich üblicherweise die olefinische Verbindung, die erzeugten Aldehyde sowie geringe Anteile der wäßrigen Phase, während die wäßrige Phase üblicherweise Rhodium, die sulfonierten Triarylphosphine, die Verbindung der Formel (1), Wasser und geringe Anteile der organischen Phase enthält.

Das Hydroformylierungsgemisch wird nach Abschluß der Umsetzung durch Druckentspannung von Kohlenmonoxid und Wasserstoff befreit und das Reaktionsprodukt, gegebenenfalls nach Abkühlen, von der den Katalysator und die Verbindung der Formel (1) enthaltenden wäßrigen Phase durch Phasentrennung abgetrennt.

Die den Katalysator und die Verbindung der Formel (1) enthaltende wäßrige Phase kann wieder in das erfindungsgemäße Verfahren zurückgeführt werden, während die abgetrennte, das Reaktionsprodukt enthaltende organische Phase, beispielsweise durch fraktionierte Destillation, aufgearbeitet wird.

Das Verfahren läßt sich kontinuierlich und diskontinuierlich durchführen.

Die nachfolgenden Beispiele beschreiben die Erfindung näher, ohne sie zu beschränken.

### Experimenteller Teil:

### Hydroformylierung von Propen

### 1. Beschreibung der Versuchsapparatur

Die für die kontinuierliche Hydroformylierung von Propen verwendete Reaktionsapparatur besteht aus einem Reaktor (Volumen 11), einem dem Reaktor nachgeschaltetem Hochdruckabscheider und einem dem Hochdruckabscheider nachgeschalteten Phasentrenngefäß. Während der Hydroformylierung befinden sich im Reaktor wäßrige Katalysatorlösung, nicht umgesetztes Propen, Reaktionsprodukte und Synthesegas. Ein im Reaktor eingebauter Rührer sorgt für eine gute Durchmischung.

Über ein in den Reaktor hineinragendes Tauchrohr werden Propen und Wasser zudosiert. Die Wasserzugabe dient dazu, die Wasseranteile, die mit dem Hydroformylierungsprodukt ausgetragen und der wäßrigen Katalysatorlösung entzogen werden, zu ergänzen. Über ein in den Reaktor eintauchendes Tauchrohr wird das Reaktionsgemisch aus dem Reaktor entfernt und einem Hochdruckabscheider zugeführt. Im Hochdruckabscheider erfolgt die Trennung des Reaktionsgemisches in gasförmige und flüssige Bestandteile. Die gasförmigen, im wesentlichen nichtumgesetztes Synthesegas, geringe Mengen Propen und Reaktionsprodukte enhaltenden Bestandteile werden aus dem Hochdruckabscheider abgeführt und in einem nachgeschalteten weiteren Abscheider nach Kühlung von den organischen Produkten abgetrennt. Das von den organischen Anteilen befreite nicht umgesetzte Synthesegas wird nach Rekomprimierung der Reaktion wieder zugeführt.

Die flüssigen, im wesentlichen die wäßrige Katalysatorlösung und das Reaktionsgemisch enthaltenden Bestandteile werden aus dem Hochdruckabscheider entfernt und dem dem Hochdruckabscheider nachgeschalteten Phasentrenngefäß zugeführt. Im Phasentrenngefäß kommt es zu einer Trennung von Reaktionsprodukt und wäßriger Katalysatorlösung. Das die obere Phase bildende Reaktionsprodukt wird abgetrennt und anschließend destilliert. Die untere, die wäßrige Katalysatorlösung enthaltende Phase, wird aus dem Phasentrenngefäß abgeleitet und mittels einer Pumpe dem Reaktor wieder zugeführt. Auf diese Weise wird die wäßrige Katalysatorlösung im Kreis geführt.

### 2. Versuchsdurchführung

### Beispiel

### 2.a) (Vergleichsversuch ohne Zusatz von Polyethylenglykol 400)

Die wäßrige Katalysatorlösung enthält 200 ppm Rhodium, Trinatrium-tri-(m-sulfophenylphosphin) (Na-TPPPTS) und Rhodium im Molverhältnis 100:1. Sie wird hergestellt, indem die entsprechende Menge Rhodium(III)acetat in einer wäßrigen Na-TPPTS-Lösung aufgelöst und die Katalysatorlösung unter den Bedingungen der Hydroformylierung in Gegenwart von Synthesegas (CO/H₂ = 1/1) bei 122°C präformiert wird. Der mit einem Rührwerk bestückte Reaktor (Volumen 1 l) hat im Betriebszustand einen Füllstand von 65 Volumen-% Katalysatorlösung (650 ml). Die gesamte Katalysatorlösung beträgt 850 ml, d.h. 200 ml Katalysatorlösung befinden sich in dem sich an den Reaktor anschließenden Kreislaufsystem (Hochdruckabscheider, Phasentrenngefäß und Leitungen). Es werden dem Reaktor 83,5 g Propen pro Stunde sowie 0,0955 Normkubikmeter Synthesegas kontinuierlich zugeleitet. Der Druck beträgt 50 bar und die Reaktionstemperatur 122°C. Der Reaktorinhalt wird mit Hilfe der Rührers kräftig durchmischt. Die mittlere Verweilzeit der Katalysatorlösung beträgt 0,43 h⁻¹. Die im Phasentrenngefäß abgetrennte Katalysatorlösung (1,5 l/h) wird dem Reaktor wieder zugeführt.

Der Propenumsatz beträgt 90 %. Das entspricht einer Produktivität von 0,2 kg Rohhydroformylierungsprodukt pro I Katalysatorlösung und Stunde (0,2 kg/(l Kat x h). Das Verhältnis von n-Butyraldehyd zu 2-Methylpropanal beträgt 93:7.

### Beispiel

### 2.b) Hydroformylierung von Propen unter Zusatz von Polyethylenglykol 400

Es wird wie im Vergleichsversuch 2.a) beschrieben gearbeitet, jedoch mit dem Unterschied, daß die wäßrige Katalysatorlösung 9,5 Gew.-% Polyethylenglykol mit einem mittleren Molekulargewicht von 400 (PEG 400) enthält, der Propeneinsatz auf 100g/h und die Synthesegasmenge auf 0,102 Normkubikmeter pro Stunde erhöht wird.

Der Propenumsatz beträgt nun 95 % bis 96 %. Das entspricht einer Produktivität von 0,25 kg Rohhydroformylierungsprodukt pro l Katalysatorlösung und Stunde (0,25 kg/(I Kat x h). Das Verhältnis von n-Butyraldehyd zu 2-Methylpropanal beträgt 91:9.

## Patentansprüche

1. Verfahren zur Herstellung von Aldehyden, **dadurch gekennzeichnet, daß** man Propen in Anwesenheit einer Rhodium und sulfonierte Triarylphosphine als Katalysator und von 1 bis 15 Gew.-% einer Verbindung der Formel (1) R(OCH₂CH₂)ₙOR¹ enthaltenden wäßrigen Phase, wobei die Triarlyphosphine wenigstens drei -(SO₃)M-Reste enthalten, bei denen M gleich oder verschieden ist und M für H, ein Alkalimetallion, ein Ammoniumion, ein quatemäres Ammoniumion, ein ½ Erdalkalimetallion oder ½ Zinkion steht, und wobei in der Formel (1) R für Wasserstoff, einen geradkettigen oder verzweigten Alkylrest mit 1 bis 4 Kohlenstoffatomen oder einen Hydroxyalkylrest mit 1 bis 4 Kohlenstoffatomen, R¹ für Wasserstoff oder einen Methylrest und n für eine ganze Zahl von 3 bis 50 steht, mit Wasserstoff und Kohlenmonoxid bei 20 bis 170°C und 1 bis 300 bar umsetzt.

2. Verfahren nach Anspruch 1, **dadurch gekennzeichnet, daß** man als sulfonierte Triarylphosphine Verbindungen der Formel (2) 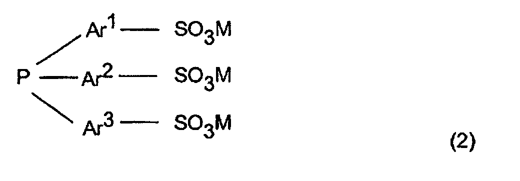 worin Ar¹, Ar² und Ar³ gleich oder verschieden sind und jeweils einen Phenyloder Naphthylrest bedeuten, und M gleich oder verschieden ist und für ein Alkalimetallion, ein Ammoniumion, ein quatemäres Ammoniumion oder ein ½ Erdalkalimetallion oder ½ Zinkion steht, einsetzt.

3. Verfahren nach Anspruch 1 oder 2, **dadurch gekennzeichnet, daß** man als sulfonierte Triarylphosphine ein trisulfoniertes Triphenylphosphin einsetzt.

4. Verfahren nach Anspruch 1 oder 2, **dadurch gekennzeichnet, daß** man als sulfoniertes Triarylphosphin Trinatrium-tri(m-sulfophenyl)phosphin einsetzt.

5. Verfahren nach einem oder mehreren der Ansprüche 1 bis 4, **dadurch gekennzeichnet, daß** man die wäßrige Phase entsprechend einer Menge von 2x10⁻⁶ bis 5x10⁻² Mol Rhodium pro Mol olefinische Verbindung einsetzt.

6. Verfahren nach einem oder mehreren der Ansprüche 1 bis 5, **dadurch gekennzeichnet, daß** man Rhodium und sulfonierte Triarylphosphine der Formel (2) im Molverhältnis 1 : 10 bis 1 : 1000 einsetzt.

7. Verfahren nach einem oder mehreren der Ansprüche 1 bis 6, **dadurch gekennzeichnet, daß** man Rhodium und sulfonierte Triarylphosphine der Formel (2) im Molverhältnis 1 : 50 bis 1 : 200 einsetzt.

8. Verfahren nach einem oder mehreren der Ansprüche 1 bis 7, **dadurch gekennzeichnet, daß** die wäßrige Phase bei Verwendung sulfonierter Triarylphosphine der Formel (2) 100 bis 1000 ppm Rhodium enthält.

9. Verfahren nach einem oder mehreren der Ansprüche 1 bis 8, **dadurch gekennzeichnet, daß** die wäßrige Phase bei Verwendung sulfonierter Triarylphosphine der Formel (2) 200 bis 500 ppm Rhodium enthält.

10. Verfahren nach einem oder mehreren der Ansprüche 1 bis 9, **dadurch gekennzeichnet, daß** die wäßrige Phase bei Verwendung sulfonierter Triarylphosphine der Formel (2) 300 bis 400 ppm Rhodium enthält.

11. Verfahren nach einem oder mehreren der Ansprüche 1 bis 10, **dadurch gekennzeichnet, daß** man als sulfonierte Triarylphosphine Verbindungen der Formel (3) 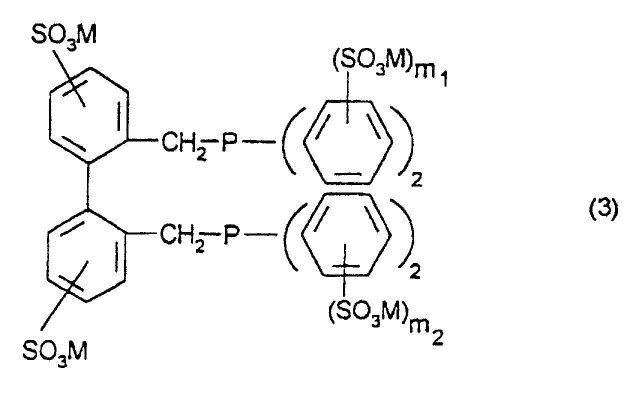 einsetzt, worin m₁ und m₂ unabhängig voneinander für 0 oder 1 steht und die Verbindungen der Formel (3) drei bis sechs -SO₃M Gruppen, worin M die vorstehend genannte Bedeutung besitzt, enthalten.

12. Verfahren nach einem oder mehreren der Ansprüche 1 bis 11, **dadurch gekennzeichnet, daß** man als sulfonierte Triarylphosphine Verbindungen der Formel (4) 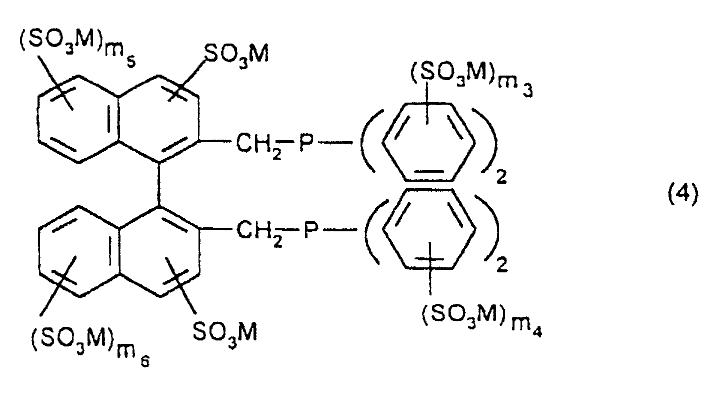 einsetzt, worin m₃, m₄, m₅ und m₆ unabhängig voneinander für 0 oder 1 steht und die Verbindungen der Formel (4) vier bis acht -SO₃M Gruppen, worin M die vorstehend genannten Bedeutung besitzt, enthalten.

13. Verfahren nach einem oder mehreren der Ansprüche 1 bis 12, **dadurch gekennzeichnet, daß** man Rhodium und sulfonierte Triarylphosphine der Formel (3) oder (4) im Molverhältnis 1:5 bis 1:100 einsetzt.

14. Verfahren nach einem oder mehreren der Ansprüche 1 bis 13, **dadurch gekennzeichnet, daß** man Rhodium und sulfonierte Triarylphosphine der Formel (3) und (4) im Molverhältnis 1:5 bis 1:50 einsetzt.

15. Verfahren nach einem oder mehreren der Ansprüche 1 bis 14, **dadurch gekennzeichnet, daß** man Rhodium und sulfonierte Triarylphosphine der Formel (3) oder (4) im Molverhältnis 1:8 bis 1:15 einsetzt.

16. Verfahren nach einem oder mehreren der Ansprüche 1 bis 15, **dadurch gekennzeichnet, daß** die wäßrige Phase bei Verwendung sulfonierter Triarylphosphine der Formel (3) oder (4) 20 bis 500 ppm Rhodium enthält.

17. Verfahren nach einem oder mehreren der Ansprüche 1 bis 16, **dadurch gekennzeichnet, daß** die wäßrige Phase bei Verwendung sulfonierter Triarylphosphine der Formel (3) oder (4) 30 bis 150 ppm Rhodium enthält.

18. Verfahren nach einem oder mehreren der Ansprüche 1 bis 17, **dadurch gekennzeichnet, daß** die wäßrige Phase bei Verwendung sulfonierter Triarylphosphine der Formel (3) oder (4) 40 bis 100 ppm Rhodium enthält.

19. Verfahren nach einem oder mehreren der Ansprüche 1 bis 18, **dadurch gekennzeichnet, daß** man Propen als olefinische Verbindung in Anwesenheit einer 3 bis 10 Gew.-% der Verbindung der Formel (1) enthaltenden wäßrigen Phase umsetzt.

20. Verfahren nach einem oder mehreren der Ansprüche 1 bis 19, **dadurch gekennzeichnet, daß** man ein Polyethylenglykol der Formel H(OCH₂CH₂)ₙOH, worin n für eine ganze Zahl von 3 bis 50 steht, als Verbindung der Formel (1), einsetzt.

21. Verfahren nach einem oder mehreren der Ansprüche 1 bis 20, **dadurch gekennzeichnet, daß** man ein Polyethylenglykol der Formel H(OCH₂CH₂)ₙOH, worin n für eine ganze Zahl von 4 bis 30 steht, als Verbindung der Formel (1) einsetzt.

22. Verfahren nach einem oder mehreren der Ansprüche 1 bis 21, **dadurch gekennzeichnet, daß** man ein Polyethylenglykol der Formel H(OCH₂CH₂)ₙOH, worin n für eine ganze Zahl von 6 bis 12 steht, als Verbindung der Formel (1) einsetzt.

23. Verfahren nach einem oder mehreren der Ansprüche 1 bis 22, **dadurch gekennzeichnet, daß** man eine Verbindung der Formel R(OCH₂CH₂)ₙOH, worin R für einen Methylrest oder β-Hydroxypropylrest und n für eine ganze Zahl von 3 bis 50 steht, als Verbindungen der Formel (1) einsetzt.

24. Verfahren nach einem oder mehreren der Ansprüche 1 bis 23, **dadurch gekennzeichnet, daß** man eine Verbindung der Formel R(OCH₂CH₂)ₙOH, worin R für einen Methylrest oder β-Hydroxypropylrest und n für eine ganze Zahl von 4 bis 30 steht, als Verbindungen der Formel (1) einsetzt.

25. Verfahren nach einem oder mehreren der Ansprüche 1 bis 24, **dadurch gekennzeichnet, daß** man die Umsetzung bei 50 bis 150°C durchführt.

26. Verfahren nach einem oder mehreren der Ansprüche 1 bis 25, **dadurch gekennzeichnet, daß** man die Umsetzung bei 100 bis 140°C durchführt.

27. Verfahren nach einem oder mehreren der Ansprüche 1 bis 26, **dadurch gekennzeichnet, daß** man die Umsetzung bei 20 bis 150 bar durchführt.

28. Verfahren nach einem oder mehreren der Ansprüche 1 bis 27, **dadurch gekennzeichnet, daß** man die Umsetzung bei 30 bis 80 bar durchführt.

## Claims

1. A process for preparing aldehydes, which comprises reacting propylene in the presence of an aqueous phase comprising rhodium and sulfonated triarylphosphines as catalyst and from 1 to 15% by weight of a compound of the formula (1) R(OCH₂CH₂)ₙOR, where the triarylphosphines contain at least three -(SO₃)M-groups, in which M are identical or different and are each hydrogen, an alkali metal ion, an ammonium ion, a quaternary ammonium ion, an ½ alkaline earth metal ion or ½ zinc ion, and where, in the formula (1), R is hydrogen, a straight-chain or branched alkyl radical having from 1 to 4 carbon atoms or a hydroxyalkyl radical having from 1 to 4 carbon atoms, R¹ is hydrogen or a methyl radical and n is an integer from 3 to 50, with hydrogen and carbon monoxide at from 20 to 170°C and from 1 to 300 bar.

2. The process as claimed in claim 1, wherein the sulfonated triarylphosphines used are compounds of the formula (2) 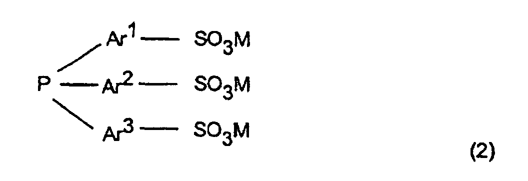 where Ar¹, Ar² and Ar³ are identical or different and are each a phenyl or naphthyl radical and M are identical or different and are each an alkali metal ion, an ammonium ion, a quaternary ammonium ion or a ½ alkaline earth metal ion or ½ zinc ion.

3. The process as claimed in claim 1 or 2, wherein the sulfonated triarylphosphine used is a trisulfonated triphenylphosphine.

4. The process as claimed in claim 1 or 2, wherein the sulfonated triarylphosphine used is trisodium tri(m-sulfophenyl)phosphine.

5. The process as claimed in one or more of claims 1 to 4, wherein the aqueous phase is used in an amount corresponding to from 2 x 10⁻⁶ to 5 x 10⁻² mol of rhodium per mol of olefinic compound.

6. The process as claimed in one or more of claims 1 to 5, wherein rhodium and sulfonated triarylphosphines of the formula (2) are used in a molar ratio of from 1:10 to 1:1000.

7. The process as claimed in one or more of claims 1 to 6, wherein rhodium and sulfonated triarylphosphines of the formula (2) are used in a molar ratio of from 1:50 to 1:200.

8. The process as claimed in one or more of claims 1 to 7, wherein the aqueous phase contains from 100 to 1000 ppm of rhodium when using sulfonated triarylphosphines of the formula (2).

9. The process as claimed in one or more of claims 1 to 8, wherein the aqueous phase contains from 200 to 500 ppm of rhodium when using sulfonated triarylphosphines of the formula (2).

10. The process as claimed in one or more of claims 1 to 9, wherein the aqueous phase contains from 300 to 400 ppm of rhodium when using sulfonated triarylphosphines of the formula (2).

11. The process as claimed in one or more of claims 1 to 10, wherein the sulfonated triarylphosphines used are compounds of the formula (3) 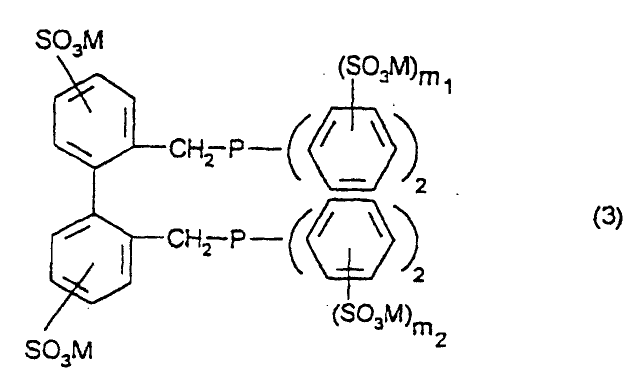 where m₁ and m₂ are, independently of one another, 0 or 1 and the compounds of the formula (3) contain from three to six -SO₃M groups, where M is as defined above.

12. The process as claimed in one or more of claims 1 to 11, wherein the sulfonated triarylphosphines used are compounds of the formula (4) 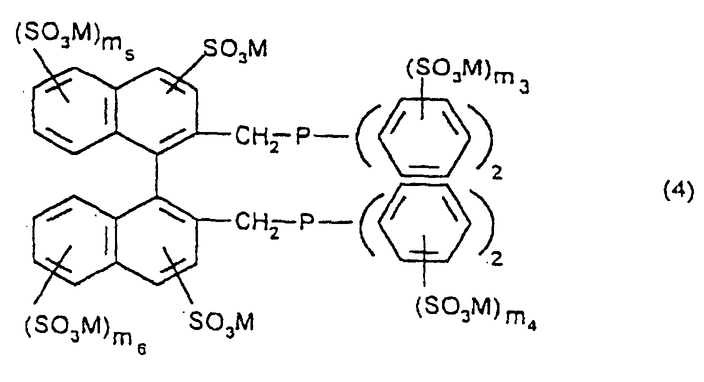 where m₃, m₄, m₅ and m₆ are, independently of one another, 0 or 1 and the compounds of the formula (4) have from four to eight -SO₃M groups, where M is as defined above.

13. The process as claimed in one or more of claims 1 to 12, wherein rhodium and sulfonated triarylphosphines of the formula (3) or (4) are used in a molar ratio of 1:5 to 1:100.

14. The process as claimed in one or more of claims 1 to 13, wherein rhodium and sulfonated triarylphosphines of the formula (3) or (4) are used in a molar ratio of 1:5 to 1:50.

15. The process as claimed in one or more of claims 1 to 14, wherein rhodium and sulfonated triarylphosphines of the formula (3) or (4) are used in a molar ratio of 1:8 to 1:15.

16. The process as claimed in one or more of claims 1 to 15, wherein the aqueous phase contains from 20 to 500 ppm of rhodium when using sulfonated triarylphosphines of the formula (3) or (4).

17. The process as claimed in one or more of claims 1 to 16, wherein the aqueous phase contains from 30 to 150 ppm of rhodium when using sulfonated triarylphosphines of the formula (3) or (4).

18. The process as claimed in one or more of claims 1 to 17, wherein the aqueous phase contains from 40 to 100 ppm of rhodium when using sulfonated triarylphosphines of the formula (3) or (4).

19. The process as claimed in one or more of claims 1 to 18, wherein propene as olefinic compound is reacted in the presence of an aqueous phase containing from 3 to 10% by weight of the compound of the formula (1).

20. The process as claimed in one or more of claims 1 to 19, wherein the compound of the formula (1) used is a polyethylene glycol of the formula H(OCH₂CH₂)ₙOH, where n is an integer from 3 to 50.

21. The process as claimed in one or more of claims 1 to 20, wherein the compound of the formula (1) used is a polyethylene glycol of the formula H(OCH₂CH₂)ₙOH, where n is an integer from 4 to 30.

22. The process as claimed in one or more of claims 1 to 21, wherein the compound of the formula (1) used is a polyethylene glycol of the formula H(OCH₂CH₂)ₙOH, where n is an integer from 6 to 12.

23. The process as claimed in one or more of claims 1 to 22, wherein the compound of the formula (1) used is a compound of the formula R(OCH₂CH₂)ₙOH, where R is a methyl radical or β-hydroxypropyl radical and n is an integer from 3 to 50.

24. The process as claimed in one or more of claims 1 to 23, wherein the compound of the formula (1) used is a compound of the formula R(OCH₂CH₂)ₙOH, where R is a methyl radical or β-hydroxypropyl radical and n is an integer from 4 to 30.

25. The process as claimed in one or more of claims 1 to 24, wherein the reaction is carried out at from 50 to 150°C.

26. The process as claimed in one or more of claims 1 to 25, wherein the reaction is carried out at from 100 to 140°C.

27. The process as claimed in one or more of claims 1 to 26, wherein the reaction is carried out at from 20 to 150 bar.

28. The process as claimed in one or more of claims 1 to 27, wherein the reaction is carried out at from 30 to 80 bar.

## Revendications

1. Procédé pour la préparation d'aldéhydes, **caractérisé en ce qu'**on transforme du propylène en présence d'une phase aqueuse contenant du rhodium et des triarylphosphines sulfonées comme catalyseur et 1 à 15% en poids d'un composé de formule (1) R(OCH₂CH₂)ₙOR¹, les triarylphosphines contenant au moins trois radicaux -(SO₃)M, dans lesquels M est identique ou différent et M représente H, un ion de métal alcalin, un ion d'ammonium, un ion d'ammonium quaternaire, un 1/2 ion de métal alcalino-terreux ou 1/2 ion de zinc, et, dans la formule (1), R représentant un hydrogène, un radical alkyle linéaire ou ramifié comprenant 1 à 4 atomes de carbone ou un radical hydroxyalkyle comprenant 1 à 4 atomes de carbone, R¹ représentant un hydrogène ou un radical méthyle et n représentant un nombre entier de 3 à 50, avec de l'hydrogène et du monoxyde de carbone à 20 jusqu'à 170°C et 1 à 300 bars.

2. Procédé selon la revendication 1, **caractérisé en ce qu'**on utilise comme triarylphosphines sulfonées des composés de formule (2) 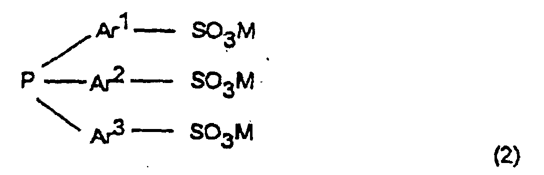 dans laquelle Ar¹, Ar² et Ar³ sont identiques ou différents et signifient à chaque fois un radical phényle ou naphtyle, et M est identique ou différent et représente un ion de métal alcalin, un ion d'ammonium, un ion d'ammonium quaternaire ou un 1/2 ion de métal alcalino-terreux ou un 1/2 ion de zinc.

3. Procédé selon les revendications 1 ou 2, **caractérisé en ce qu'**on utilise une triphénylphosphine trisulfonée comme triarylphosphines sulfonées.

4. Procédé selon les revendications 1 ou 2, **caractérisé en ce qu'**on utilise de la tri(m-sulfophényl)phosphine trisodique comme triarylphosphine sulfonée.

5. Procédé selon l'une quelconque ou plusieurs des revendications 1 à 4, **caractérisé en ce qu'**on utilise la phase aqueuse correspondant à une quantité de 2x10⁻⁶ à 5x10⁻² mole de rhodium par mole de composé oléfinique.

6. Procédé selon l'une quelconque ou plusieurs des revendications 1 à 5, **caractérisé en ce qu'**on utilise le rhodium et les triarylphosphines sulfonées de formule (2) dans un rapport molaire de 1:10 à 1:1000.

7. Procédé selon l'une quelconque ou plusieurs des revendications 1 à 6, **caractérisé en ce qu'**on utilise le rhodium et les triarylphosphines sulfonées de formule (2) dans un rapport molaire de 1:50 à 1:200.

8. Procédé selon l'une quelconque ou plusieurs des revendications 1 à 7, **caractérisé en ce que** la phase aqueuse contient 100 à 1000 ppm de rhodium dans le cas de l'utilisation de triarylphosphines sulfonées de formule (2).

9. Procédé selon l'une quelconque ou plusieurs des revendications 1 à 8, **caractérisé en ce que** la phase aqueuse contient 200 à 500 ppm de rhodium dans le cas de l'utilisation de triarylphosphines sulfonées de formule (2).

10. Procédé selon l'une quelconque ou plusieurs des revendications 1 à 9, **caractérisé en ce que** la phase aqueuse contient 300 à 400 ppm de rhodium dans le cas de l'utilisation de triarylphosphines sulfonées de formule (2).

11. Procédé selon l'une quelconque ou plusieurs des revendications 1 à 10, **caractérisé en ce qu'**on utilise comme triarylphosphines sulfonées des composés de formule (3) 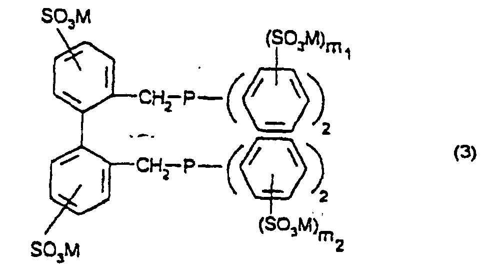 dans laquelle m₁ et m₂ représentent, indépendamment l'un de l'autre, 0 ou 1 et les composés de formule (3) présentent trois à six groupements -SO₃M, M présentant la signification susmentionnée.

12. Procédé selon l'une quelconque ou plusieurs des revendications 1 à 11, **caractérisé en ce qu'**on utilise comme triarytphosphines sulfonées des composés de formule (4) 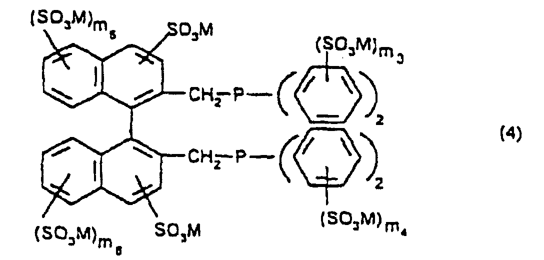 dans laquelle m₃, m₄, m₅ et m₆ représentent, indépendamment l'un de l'autre, 0 ou 1 et les composés de formule (4) présentent quatre à huit groupements -SO₃M, M présentant la signification susmentionnée.

13. Procédé selon l'une quelconque ou plusieurs des revendications 1 à 12, **caractérisé en ce qu'**on utilise le rhodium et les triarylphosphines sulfonées de formule (3) ou (4) dans un rapport molaire de 1:5 à 1:100.

14. Procédé selon l'une quelconque ou plusieurs des revendications 1 à 13, **caractérisé en ce qu'**on utilise le rhodium et les triarylphosphines sulfonées de formule (3) ou (4) dans un rapport molaire de 1:5 à 1:50.

15. Procédé selon l'une quelconque ou plusieurs des revendications 1 à 14, **caractérisé en ce qu'**on utilise le rhodium et les triarylphosphines sutfonées de formule (3) ou (4) dans un rapport molaire de 1:8 à 1:15.

16. Procédé selon l'une quelconque ou plusieurs des revendications 1 à 15, **caractérisé en ce que** la phase aqueuse contient 20 à 500 ppm de rhodium dans le cas de l'utilisation de triarylphosphines sulfonées de formule (3) ou (4).

17. Procédé selon l'une quelconque ou plusieurs des revendications 1 à 16, **caractérisé en ce que** la phase aqueuse contient 30 à 150 ppm de rhodium dans le cas de l'utilisation de triarylphosphines sulfonées de formule (3) où (4).

18. Procédé selon l'une quelconque ou plusieurs des revendications 1 à 17, **caractérisé en ce que** la phase aqueuse contient 40 à 100 ppm de rhodium dans le cas de l'utilisation de triarylphosphines sulfonées de formule (3) ou (4).

19. Procédé selon l'une quelconque ou plusieurs des revendications 1 à 18, **caractérisé en ce qu'**on transforme du propylène comme composé oléfinique en présence d'une phase aqueuse contenant 3 à 10% en poids du composé de formule (1).

20. Procédé selon l'une quelconque ou plusieurs des revendications 1 à 19, **caractérisé en ce qu'**on utilise un polyéthylèneglycot de formule H(OCH₂CH₂)ₙOH dans laquelle n représente un nombre entier de 3 a 50 comme composé de formule (1).

21. Procédé selon l'une quelconque ou plusieurs des revendications 1 à 20, **caractérisé en ce qu'**on utilise un polyétnylèneglycol de formule H(OCH₂CH₂)ₙOH dans laquelle n représente un nombre entier de 4 à 30 comme composé de formule (1).

22. Procédé selon l'une quelconque ou plusieurs des revendications 1 à 21, **caractérisé en ce qu'**on utilise un polyéthylèneglycol de formule H(OCH₂CH₂)ₙOH dans laquelle n représente un nombre entier de 6 à 12 comme composé de formule (1).

23. Procédé selon l'une quelconque ou plusieurs des revendications 1 à 22, **caractérisé en ce qu'**on utilise un composé de formule R(OCH₂CH₂)ₙOH dans laquelle R représente un radical méthyle ou un radical β-hydroxypropyle et n représente un nombre entier de 3 à 50 comme composés de formule (1).

24. Procédé selon l'une quelconque ou plusieurs des revendications 1 à 23, **caractérisé en ce qu'**on utilise un composé de formule R(OCH₂CH₂)ₙOH dans laquelle R représente un radical méthyle ou un radical β-hydroxypropyle et n représente un nombre entier de 4 à 30 comme composés de formule (1).

25. Procédé selon l'une quelconque ou plusieurs des revendications 1 à 24, **caractérisé en ce qu'**on effectue la transformation à 50 jusqu'à 150°C,

26. Procédé selon l'une quelconque ou plusieurs des revendications 1 à 25, **caractérisé en ce qu'**on effectue la transformation à 100 jusqu'à 140°C.

27. Procédé selon l'une quelconque ou plusieurs des revendications 1 à 26, **caractérisé en ce qu'**on effectue la transformation à 20 jusqu'à 150 bars.

28. Procédé selon l'une quelconque ou plusieurs des revendications 1 à 27, **caractérisé en ce qu'**on effectue la transformation à 30 jusqu'à 80 bars.
